# EUROPEAN PATENT SPECIFICATION

(11) **EP 1 260 182 B1**
(45) Date of publication and mention of the grant of the patent: **28.11.2007**
(21) Application number: 02253427.5
(22) Date of filing: 16.05.2002
(51) Int. Cl.: A61B 17/04

(54) **Threaded suture anchor**
Chirurgischer Nähfadenanker mit Gewinde
Ancrage de suture vissé

(30) Priority: 17.05.2001 US 860059
(43) Date of publication of application: 27.11.2002
(73) Proprietor: ETHICON, INC., Somerville, NJ 08876 (US)
(72) Inventor: Justin, Dan, Logan, UT 84321 (US); Hays, Greta Jo, Logan, UT 84341 (US); Jensen, Keneth L., Providence, UT 84332 (US)
(74) Representative: Fisher, Adrian John

(56) References cited:
- WO-A-96/25887
- WO-A-96/41573
- US-A- 5 904 704
- US-A- 5 964 783
- US-B1- 6 214 031

## Description

### Technical Field

The field of art to which this invention relates is orthopedic devices, more specifically, suture anchor devices for anchoring soft tissue to bone.

### Background of the Invention

The field of sports medicine has made significant progress over the past decade. New arthroscopic techniques and soft tissue repair and attachment devices have been developed that provide for superior repair of soft tissue in joints, while minimizing recovery time. In a typical arthroscopic surgical procedure, several small incisions are made through the patient's skin in the area surrounding the injury to a joint. The joint is insufflated with a fluid such as sterile saline and an arthroscope is inserted into the joint so that the surgeon may view the operative site remotely. The surgeon then introduces specifically designed, minimally invasive arthroscopic instruments through cannulas or through other openings to perform the arthroscopic procedure. Typically, an arthroscopic procedure is a soft tissue repair procedure involving the attachment of soft tissue such as tendons, ligaments or cartilage to bone, or soft tissue to soft tissue as in cartilage repair. To facilitate the attachment of soft tissue to bone, suture anchor devices have been developed and are commonly used. A suture anchor typically consists of a mechanical fixation section, such as a threaded member or a barbed member or an irregularly shaped member such as a wedge, which is inserted into bone and is firmly engaged by the bone and fixed in place. The suture anchor also has a suture mounting section for mounting surgical sutures, which are then used to engage soft tissue and mount it to the surface of the bone adjacent to the suture anchor, in manner effective to facilitate reattachment of the soft tissue to the bone.

One common orthopedic sports medicine procedure is the repair of the rotator cuff in the shoulder. The rotator cuff is composed of four tendons that blend together and work with the deltoid to elevate, rotate and help stabilize the arm. These tendons connect the supraspinatus, infraspinatus, teres minor, and subscapularis muscles to the humeral head. Rotator cuff tears may involve one or all of the aforementioned muscles and essentially involve separation of their attachment onto their respective bony prominence. The most commonly involved tendon in rotator cuff tears is the supraspinatus tendon.

When a tear or rupture occurs in the rotator cuff, it is often necessary to re-attach the torn tendon or tendons to the bone of the humeral head. This procedure can be done open or arthroscopically using a minimally invasive procedure. It is typical to use conventional threaded, self-tapping suture anchors for these procedures. These anchors typically have a pointed distal end and a plurality of thread flights. In use, the surgeon engages the distal and of the anchor with the outer surface of the bone and begins to rotate the anchor. Eventually, after the distal tip has penetrated into the bone, the distal or leading end of the thread flights engages the bone and the screw moves into and engages the bone. Surgical needles and sutures mounted to the threaded anchor are used to affix the torn tendon to the surface of the bone.

US 5,964,783 discloses a suture anchor with an insert-molded suture. A drive head is disposed on the proximal end of the body, and a screw thread spirals around the body. US 5,904,704 discloses a suture anchor assembly having a drill portion, a thread portion and a suture attachment portion. WO 96/25887 discloses a suture anchor assembly comprising a pointed free end, a cutting flute, and screw threads. WO 96/41573 discloses a self-drilling bone securing component comprising a head end, a shank section with a thread and a front section with a point in the form of a drill.

Although the screw threaded anchors known in the art are effective for their intended purpose, there is a need in this art for novel screw threaded suture anchors having improved properties.

### Disclosure of the Invention

Therefore, it is an object of the present invention to provide a novel, threaded suture anchor that requires a lower force to engage bone.

Accordingly, a threaded suture anchor is disclosed. The suture anchor has a root portion having a proximal end and a distal end. The root portion is preferably tapered from the proximal end to the distal end. Extending proximally from the proximal end of the root portion is a suture-mounting member having a hole for receiving a suture. A plurality of thread flights extend from the root portion. The thread flights are preferably continuous and preferably increase in diameter from the distal end to the proximal end. Extending from the distal end of the root portion is a drill member.

Another aspect of the present invention is a method of using the above-described suture anchor in a surgical procedure to attach soft tissue to bone, preferably a rotator cuff repair procedure.

The novel anchor devices of the present invention have improved bone penetration characteristics allowing the surgeon to more readily and efficiently install the anchors in a surgical procedure.

These and other aspects and advantages of the present invention will become more apparent from the following description and accompanying drawings.

### Brief Description of the Drawings

FIG. 1 is a perspective view of a suture anchor of the present invention.
FIG. 2 is a side view of the suture anchor of FIG. 1.
FIG. 3A is an end view of the suture anchor of FIG. 1, illustrating the proximal, suture mounting end of the anchor.
FIG. 3B is an end view of the suture anchor of FIG.1, illustrating the distal end of the anchor.
FIG. 4 is a side view of a threaded suture anchor of the prior art.
FIG. 5A illustrates the suture anchors of the present invention being used to secure a rotator cuff tendon to a humeral bone in a rotator cuff repair surgical procedure.
FIG. 5B is an exploded side view of a suture anchor of FIG.5A emplaced in bone with the sutures affixing the rotator cuff tendon to the surface of the humeral bone.

### Description of the Preferred Embodiments

The suture anchors 10 of the present invention made be made from biocompatible metallic materials conventionally used for implantable medical devices such as stainless steel, cobalt-chromium alloys, titanium alloys, and the like and combinations thereof. Also, the anchors 10 may be made from bioabsorbable polymers including Poly(L-lactide), Poly(DL-lactide), Polyglycolide, 95:5 Poly(DL-lactide-co-glycolide), Polydioxanone, Polyesteramides, Copolyoxalates, Polycarbonates, Poly(glutamic-co-leucine), 90:10 Poly(DL-lactide-co-glycolide), 85:15 Poly(DL-lactide-co-glycolide), 75:25 Poly(DL-lactide-co-glycolide), 50:50 Poly(DL-lactide-co-glycolide), 90:10 Poly(DL-lactide-co-caprolactone), 75:25 Poly(DL-lactide-co-caprolactone), 50:50 Poly(DL-lactide-co-caprolactone), Polycaprolactone), and the like and combinations thereof. The anchors of the present invention may also be made from bioactive ceramics including tri-calcium phosphate, hydroxyapitite and the like and equivalents thereof, and bioabsorbable composites made from combinations of bioabsorbable polymers and bioactive ceramics and the like and equivalents thereof. Additionally, the anchors 10 can be made from allograph or autograph human tissue materials, tissue engineered xenographs from animal tissues, or processed collagenous tissues.

The anchors 10 may be made using conventional manufacturing techniques and processes including machining, casting, molding, metal injection molding, grinding, and thread rolling and the like and combinations thereof.

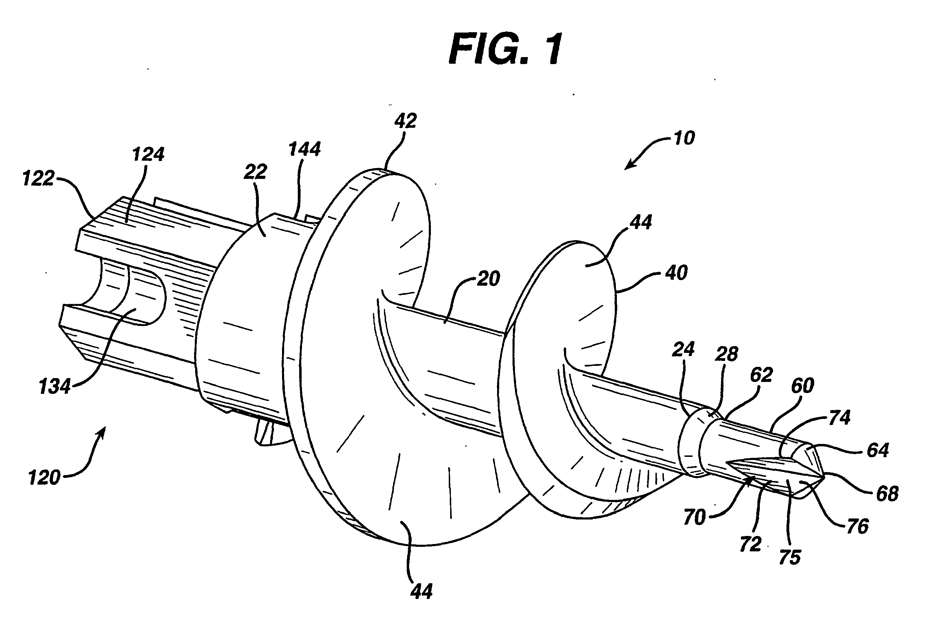

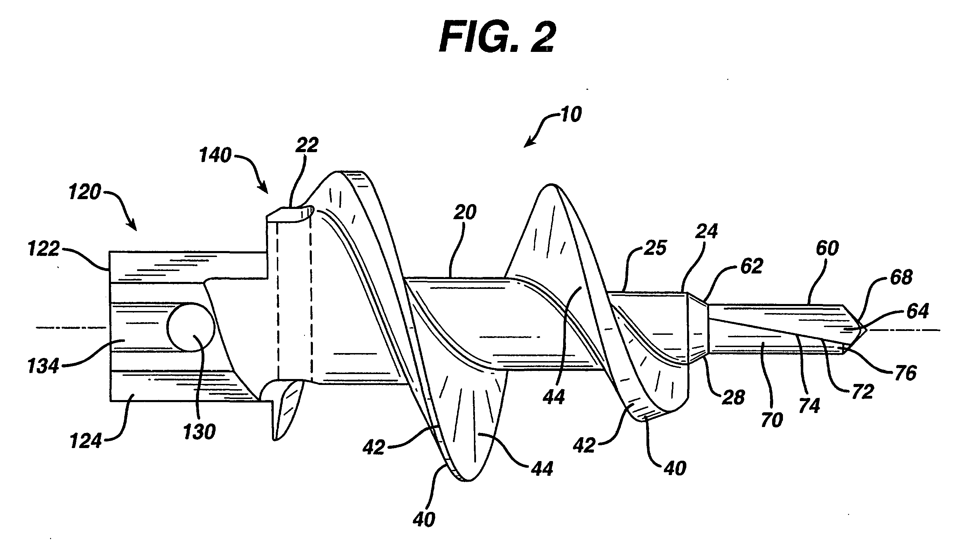

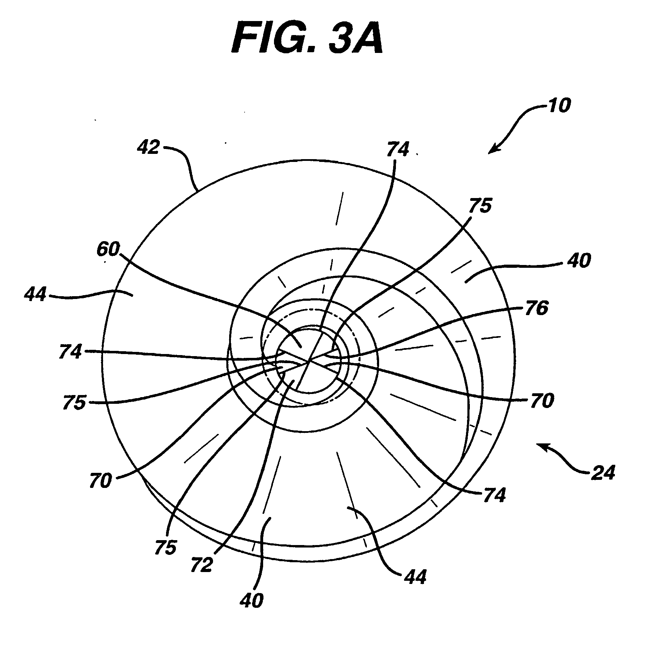

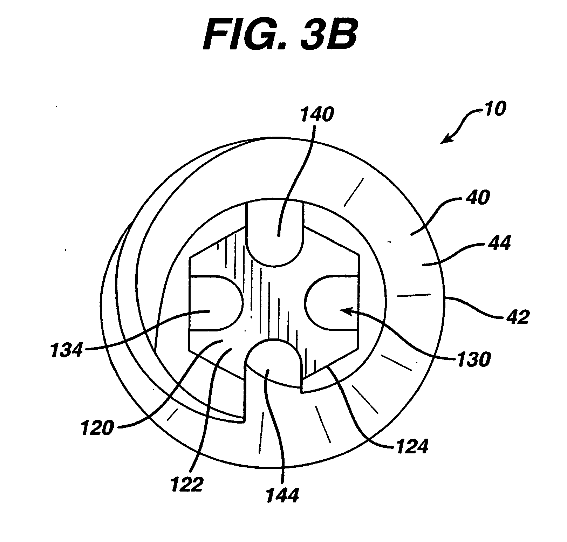

Referring now to FIGS. 1, 2, 3A and 3B, suture anchor 10 of the present invention is illustrated. The anchor 10 is seen to have a root member 20 having a proximal end 22 and a distal end 24. Root member 20 is also seen to have outer surface 25. Root member 20 preferably tapers from proximal end 22 to distal end 24. The distal end 24 is seen to have shoulder section 28. Root member 20 also preferably has a circular cross-section, although other cross-sections may be utilized although not preferred, including square, hexagonal, oval, elliptical, polygonal, and the like. Although not preferred, root member 20 may have a constant diameter. Extending outward from the surface 25 of the root member 20 are the thread flights 40. Thread flights 40 are seen to begin at proximal end 22 of root member 20 and to terminate at distal end 24. The thread flights 40 are seen to have a diameter that decreases along the length of the root member 20, having a maximum diameter at the proximal end 22 and a minimum diameter at the distal end 24. Although not preferred, the thread flights 40 may have a constant diameter along the length of root member 20. The thread flights 40 are seen to have outer edges 42 and surfaces 44. Preferably, the thread flights are continuous, but may be discontinuous. Extending from the proximal end 22 of root member 20 is the suture-mounting member 120. Suture mounting member 120 is seen to have proximal surface 122. In addition, the member 120 is seen to have a plurality of faces 124, allowing the mounting member 120 to be engaged by a conventional mechanical driving member such as wrench or a socket. Extending transversely through the member 120 is a suture-mounting hole 130. Transverse suture mounting hole 130 is seen to intersect and communicate with the suture containment slots 134. Sutures are mounted through transverse hole 130 and the suture strands are maintained within the protective slots 134 when the suture anchor is being driven into bone, and after the anchor is fully implaced in bone. Also contained in the root member 20 is the optional suture mounting hole 140. Suture mounting hole 140 is seen to be transverse to the longitudinal axis of the root member 20, and is seen to intersect and communicate with the suture containment slots 144.

Extending from the distal end 24 of root member 20 adjacent to shoulder member 28 is the drill member 60. Drill member 60 is seen to be a substantially cylindrical member of constant diameter having proximal end 62 and distal end 64. Extending from distal end 64 is the tip 68. Contained in drill member 60 is the cutting flute 70. Flute 70 is seen to have bottom 75, intersecting angulated, opposed sides 72, cutting edges 74 and opening 76 contained between sides 72. Drill member 60 is seen to have at least one cutting flute, and preferably two or more.

The anchors 10 of the present invention having sutures and surgical needles mounted thereto are typically packaged in conventional sterilizable packages and sterilized using conventional surgical techniques such as ethylene oxide sterilization, autoclaving, or radiation. The packages maintain the anchors and sutures in a sterile condition until the package is opened for use by the health care professional in a surgical procedure.

Preferably, the suture anchors 10 of the present invention have sutures mounted in the suture mounting holes prior to packaging and sterilization. The sutures may be any conventional absorbable or nonabsorbable sutures having conventional surgical needles mounted to each end of the sutures.

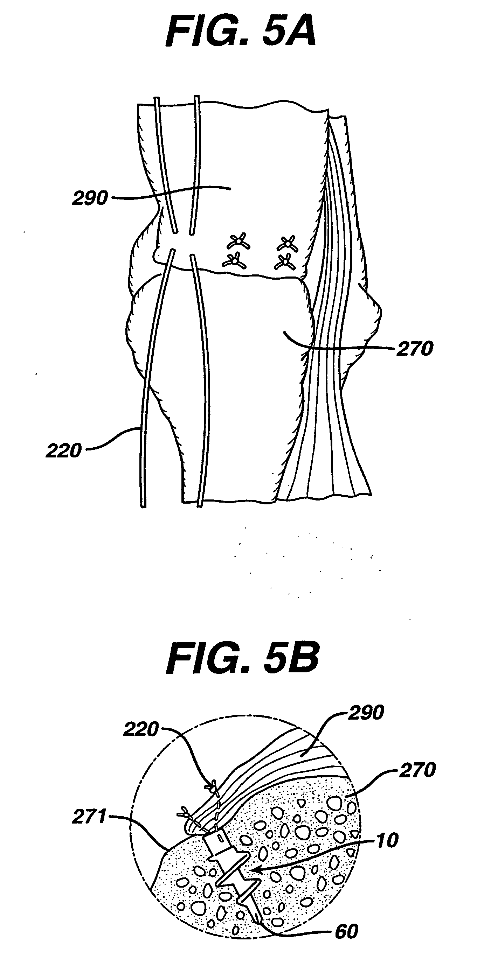

The anchors 10 of the present invention are preferably used in a conventional rotator cuff procedure, but may be used in any orthopedic and/or arthroscopic procedure in which soft tissue is attached to bone. When used in a rotator cuff repair surgical procedure, the procedure may either be open or minimally invasive utilizing arthroscopic surgical techniques. The anchors 10 of the present invention are utilized in a surgical procedure to secure soft tissue to bone in the following manner (as illustrated in FIGS. 5 A and 5B).

In a rotator cuff reattachment technique, after accessing the rotator cuff 290 and humeral head 270 in a conventional manner, the anchor 10 with suture pre-attached is secured into the head 270 of the humeral bone. This is done by contacting the surface 271 of the humeral head 270 with the tip 60 of the anchor 10, and then rotating the anchor 10 to drive the tip 60 and the remainder of the anchor 10 securely into the bone 270. The anchor 10 is mounted in a conventional driver (not shown), which is used to manipulate the anchor 10 during placement, and is then disengaged from the anchor 10 after secure placement. Then, the sutures 220 mounted to anchor 10 are passed through the cuff tissue using conventional surgical needles with the help of conventional suture passing and tissue penetrating instruments. After the suture strands 220 are passed through the tissue, they are tensioned and tied together to firmly secure and affixed the reattached rotator cuff 290 to the humeral head 270.

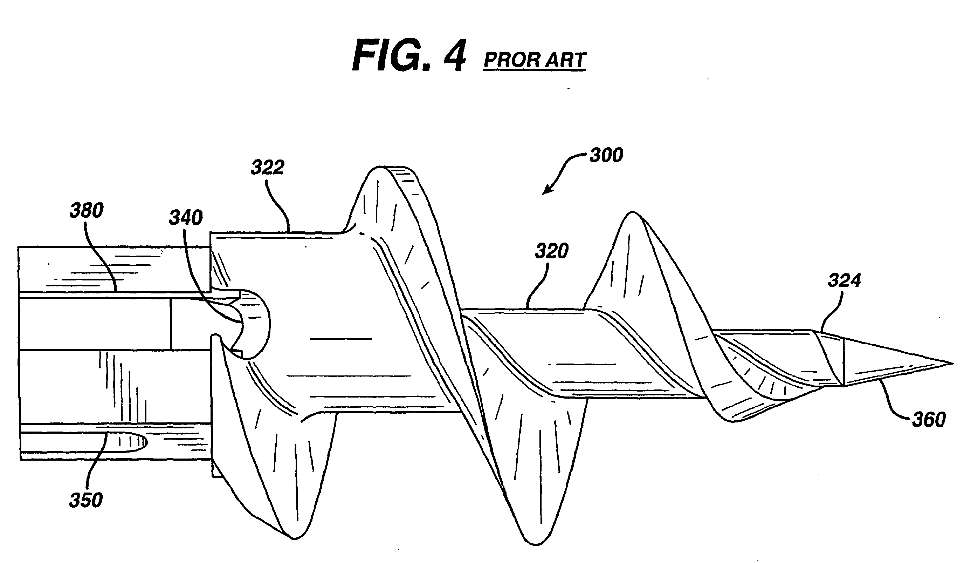

An anchor 300 of the prior art is seen in FIG. 4. The anchor 300 is seen to have a tapered root member 320 and a plurality of thread flights 340 decreasing in diameter from the proximal end 322 to the distal end 324 of the root member 320. Extending from the distal end 324 of the root member 320 is a conically tapered, pointed tip 360. Extending from the proximal end of the anchor 300 is the suture mounting drive member 380. The anchor 300 is seen to have suture mounting holes 340 and 350.

The suture anchors 10 of the present invention having a distal drill tip 60 have been found to be advantageous with regard to the force to insert when compared to the suture anchors 300 of the prior art having a conically tapered pointed tip 360. Specifically, less force is required to insert the anchors. This is important because the excessive force required to drive the anchors could result in compromised anchor fixation or fracturing of the humeral bone. Anchors are typically driven by hand without the aid of power. Preferably, surgeons should be able to feel the variations in the resistance between the anchor and the bone as the anchor is being inserted. The less torsional resistance the surgeon feels during anchor insertion, the less downward force the surgeon must apply to the anchor during insertion and the less likely he is to fracture the humeral bone.

The following example is an illustrative of the principles of practice of the present invention although not limited thereto.

### Example

A patient is prepared for arthroscopic rotator cuff surgery and anesthetized using conventional surgical procedures. Several incisions are made into the patients shoulder in the area immediately above and surrounding the patients rotator cuff in a standard manner using conventional surgical cutting instruments. The patients shoulder joint is insufflated using sterile saline.

A conventional cannula is inserted into one of the incisions and an arthroscope is placed through the cannula by the surgeon such that the distal end of the arthroscope is contained within the shoulder joint thereby illuminating the surgical site. The surgeon then utilizes a suture anchor 10 of the present invention to repair the patient's rotator cuff in the following manner.

In the first step in the cuff attachment procedure, the surgeon inserts one or more anchors 10 through cannulas with sutures attached in the greater tuberosity of the humeral head. The anchors are mounted to conventional cannulated drivers. The surgeon guides the anchor 10 and driver to the attachment site then inserts the anchor into the bone by turning the anchor 10 by hand as the distal tip 60 contacts the surface of the humeral head. As the surgeon is turning the anchor 10, the drill tip 60 on the anchor initially drills a small guide hole into the bone. Once that hole is drilled, the surgeon applies more downward force on the anchor 10 and continues to turn. This combination of torsion and compression allows the smaller diameter thread flights 40 of the anchor to engage with bone peripheral to the new hole. As the surgeon continues to turn the anchor 10, it advances further into the bone. As it advances, the larger diameter thread flights 40 engage.

The drill tip 60 in combination with the increasing diameter threads 40 allows for a smooth, controlled, anchor insertion. The surgeon continues to drive the anchor 10 into the bone until the anchor 10 is below the bone surface. He then removes the cannulated driver and leaves the anchor 10 with suture attached in the humeral head.

Using conventional suture passing instruments, needle holders, and tissue punches, the surgeon passes the free ends of the suture through the cuff tissue suing the surgical needles. The surgeon then attaches the cuff tissue to the humeral bone by tensioning and tightening the free ends of the suture down and securing, with a knot or other device, the free ends of the suture, thereby providing for a substantially fixated repair as illustrated in FIGS. 5A and 5B.

Because suture anchor 10 allows consistent attachment of sutures to bone, it can be used in other areas of orthopedic surgery including glenohumeral instability, reattachment of tendons and ligaments in the hand, elbow, foot and knee. This invention can also be applied in other surgical disciplines such as bladder suspensions in urology.

Although this invention has been shown and described with respect to detailed embodiments thereof, it will be understood by those skilled in the art that various changes in form and detail thereof may be made without departing from the scope of the claimed invention.

## Claims

1. A suture anchor(10), comprising:
an elongate root member (20) having a proximal end (22) and a distal end (24) and an outer surface(25);
a plurality of thread flights(40) extending out from the surface of the root member (20), said thread flights (40) having a pitch and each said flight (40) having a diameter, wherein the diameter of the thread flights (40) decreases along the root member (20) from the proximal end (22) to the distal end (24);
a drill member (60) extending distally from the distal end (24) of the root member (20); and
a suture mounting member (120) extending proximally from the proximal end (22) of the root member (20), **characterised in that**:
the drill member (60) is a substantially cylindrical member of constant diameter that is less than the minimum diameter of the root member (20).

2. The anchor (10) of claim 1, wherein the root member (20) has a circular cross-section.

3. The anchor (10) of claim 2, wherein the diameter of the root member (20) varies from a maximum at the proximal end (22) to a minimum at the distal end (24).

4. The anchor (10) of any preceding claim wherein the pitch of the thread flights (40) is constant.

5. The anchor (10) of any of claims 1 to 3 wherein the pitch of the thread flights (40) is variable.

6. The anchor (10) of any preceding claim wherein the drill member (60) comprises a cylindrical member having a cutting flute (70) therein.

7. The anchor (10) of any preceding claim, wherein the thread flights (40) are continuous.

8. The anchor (10) of any of claims 1 to 6, wherein the thread flights (40) are discontinuous.

9. The anchor (10) of any preceding claim, further comprising a transverse suture mounting passage (130) in the suture mounting member (120).

10. The anchor (10) of any preceding claim, additionally comprising a suture mounting passage (140) in the root member (20).

11. The anchor (10) of any preceding claim, additionally comprising a surgical suture (220) mounted thereto.

## Patentansprüche

1. Fadenanker (10), der aufweist:
ein längliches Wurzelelement (20), das ein proximales Ende (22) und ein distales Ende (24) und eine Außenfläche (25) hat;
eine Vielzahl Gewindegänge (40), die sich von der Fläche des Wurzelelementes (20) erstrecken, wobei die Gewindegänge (40) eine Steigung haben und jeder Gang (40) einen Durchmesser hat, wobei der Durchmesser der Gewindegänge (40) entlang dem Wurzelelement (20) von dem proximalen Ende (22) zu dem distalen Ende (24) abnimmt;
ein Bohrelement (60), das sich distal von dem distalen Ende (24) des Wurzelelementes (20) erstreckt; und
ein Fadeneinsetzelement (120), das sich proximal von dem proximalen Ende (22) des Wurzelelementes (20) erstreckt, **dadurch gekennzeichnet, daß**:
das Bohrelement (60) ein im wesentlichen zylindrisches Element mit konstantem Durchmesser, der geringer ist als der minimale Durchmesser des Wurzelelementes (20), ist.

2. Anker (10) nach Anspruch 1, bei dem das Wurzelelement (20) einen kreisförmigen Querschnitt hat.

3. Anker (10) nach Anspruch 2, bei dem der Durchmesser des Wurzelelementes (20) sich von einem Maximum an dem proximalen Ende (22) zu einem Minimum an dem distalen Ende (24) ändert.

4. Anker (10) nach einem der vorangehenden Ansprüche, bei dem die Steigung der Gewindegänge (40) konstant ist.

5. Anker (10) nach einem der Ansprüche 1 bis 3, bei dem die Steigung der Gewindegänge (40) variabel ist.

6. Anker (10) nach einem der vorangehenden Ansprüche, bei dem das Bohrelement (60) ein zylindrisches Element mit einer Schneidrinne (70) in diesem aufweist.

7. Anker (10) nach einem der vorangehenden Ansprüche, bei dem die Gewindegänge (40) kontinuierlich sind.

8. Anker (10) nach einem der Ansprüche 1 bis 6, bei dem die Gewindegänge (40) nicht kontinuierlich sind.

9. Anker (10) nach einem der vorangehenden Ansprüche, weiter mit einem querverlaufenden Fadeneinsetzdurchlaß (130) in dem Fadeneinsetzelement (120).

10. Anker (10) nach einem der vorangehenden Ansprüche, der zusätzlich einen Fadeneinsetzdurchlaß (140) in dem Wurzelelement (20) aufweist.

11. Anker (10) nach einem der vorangehenden Ansprüche, der zusätzlich einen daran angebrachten chirurgischen Faden (220) aufweist.

## Revendications

1. Ancrage de suture (10), comprenant :
■ un élément racine allongé (20) présentant une extrémité proximale (22) et une extrémité distale (24) et une surface externe (25) ;
■ une pluralité de filets (40) s'étendant vers l'extérieur depuis la surface de l'élément racine (20), lesdits filets (40) présentant un pas et chaque dit filet (40) présentant un diamètre, dans lequel le diamètre des filets (40) diminue le long de l'élément racine (20) de l'extrémité proximale (22) à l'extrémité distale (24) ;
■ un élément de perforation (60) s'étendant de manière distale depuis l'extrémité distale (24) de l'élément racine (20) ; et
■ un élément de montage de suture (120) s'étendant de manière proximale depuis l'extrémité proximale (22) de l'élément racine (20), **caractérisé en ce que** :
■ l'élément de perforation (60) est un élément sensiblement cylindrique de diamètre constant qui est inférieur au diamètre minimal de l'élément racine (20).

2. Ancrage (10) selon la revendication 1, dans lequel l'élément racine (20) présente une section transversale circulaire.

3. Ancrage (10) selon la revendication 2, dans lequel le diamètre de l'élément racine (20) varie d'un maximum au niveau de l'extrémité proximale (22) à un minimum au niveau de l'extrémité distale (24).

4. Ancrage (10) selon l'une quelconque des revendications précédentes dans lequel le pas des filets (40) est constant.

5. Ancrage (10) selon l'une quelconque des revendications 1 à 3 dans lequel le pas des filets (40) est variable.

6. Ancrage (10) selon l'une quelconque des revendications précédentes dans lequel l'élément de perforation (60) comprend un élément cylindrique présentant une cannelure de découpage (70) dans celui-ci.

7. Ancrage (10) selon l'une quelconque des revendications précédentes, dans lequel les filets (40) sont continus.

8. Ancrage (10) selon l'une quelconque des revendications 1 à 6, dans lequel les filets (40) sont discontinus.

9. Ancrage (10) selon l'une quelconque des revendications précédentes, comprenant en outre un passage de montage de suture transversale (130) dans l'élément de montage de suture (120).

10. Ancrage (10) selon l'une quelconque des revendications précédentes, comprenant en plus un passage de montage de suture (140) dans l'élément racine (20).

11. Ancrage (10) selon l'une quelconque des revendications précédentes, comprenant en plus une suture chirurgicale (220) montée sur celui-ci.
